# EUROPEAN PATENT APPLICATION

(11) **EP 2 634 268 A2**
(43) Date of publication of application: **04.09.2013**
(21) Application number: 13169861.5
(22) Date of filing: 07.01.2010
(51) Int. Cl.: C12Q 1/68, G01N 33/68

(54) **Enrichment and identification of fetal cells in maternal blood and ligands for such use**

(30) Priority: 07.01.2009 DK 200900020; 04.10.2009 DK 200901084
(62) Divisional of application: 10701193.4
(71) Applicant: QuantiBact A/S, 2650 Hvidovre (DK)
(72) Inventor: Christensen, Britta, 3460 Birkerød (DK); Kølvraa, Steen, 8541 Skødstrup (DK); Eckelt, Andreas, 8270 Højbjerg (DK); Brinch, Marie, 7100 Vejle (DK); Singh, Ripudaman, 8000 Aarhus C (DK); Hatt, Lotte, 8660 Skanderborg (DK)
(74) Representative: Høiberg A/S

(57) **Abstract**

The present invention relates to enrichment and/or identification of fetal cells of a maternal blood sample using fetal cell specific ligands and/or fetal cell specific hybridization probes. Enriched or identified fetal cells may be subjected to steps of detection or diagnosis, wherefore the present invention enables non-invasive prenatal diagnostics.

## Description

### Background

The examination of fetal cells for early detection of fetal diseases and genetic abnormalities is carried out in connection with many pregnancies, in particular when the maternal age is high (35 years or above) or where genetic diseases are known in the family. Fetal cells may be obtained by amniocentesis, the removal of amniotic fluid from the amniotic cavity within the amniotic sac or by chorion biopsy, where biopsies are taken from the placenta, so-called invasive sampling.

Prenatal aneuploidy screening employs either traditional chromosome analysis or chromosome specific DNA probes for elucidation of numerical aberrations of the most frequently abnormal chromosomes, particular chromosomes 13, 18, 21, X and Y in the fetus.

Due to the invasiveness of the sampling methods described above and the risk of abortion, it would be advantageously to perform fetal diagnosis by a non-invasive procedure, such as for example by use of a maternal blood sample.

During pregnancy a variety of cell types of fetal origin cross the placenta and circulate within maternal peripheral blood. The feasibility of using fetal cells in the maternal circulation for diagnostic purposes has been hindered by the fact that fetal cells are present in maternal blood in only very limited numbers, reported numbers have been from one fetal cell per 10⁵-10⁸ nucleated maternal cells or 1-10 fetal cells per ml maternal blood. In addition most fetal cells cannot be distinguished from maternal cells on the basis of morphology alone, thus alternative methods of identification of fetal cells have been investigated.

US2007/0015171 describes a non-invasive method for isolation and detection of fetal DNA. The method enriches a maternal blood sample using antibodies that bind specifically to maternal cells and/or antibodies that bind specifically to fetal cells. The inventors suggest the use of a few specifically mentioned antibodies: HLe-1 is an antibody that recognizes an antigen present on mature human leucocytes and on very immature erythrocytes precursors, but not mature nucleated erythrocytes. Thus, it is suggested that this antibody can be used to recognize maternal leucocytes, but not fetal nucleated erythrocytes. Anti-monocyte antibody (M3) and anti-lymphocyte antibody (L4) are also suggested for removing maternal cells from a sample. Finally, the authors suggest using a monoclonal antibody, which recognizes the transferrin receptor (TfR) on fetal cells. DNA from isolated fetal cells is subsequently made available for detection and diagnosis.

However, there remains a need for improved methods of isolating fetal cells from maternal blood samples such as to facilitate pre-natal detection and diagnosis.

### Summary of the invention

In a first aspect, the present invention provides a method of fixating cells of a maternal blood sample such that fetal cells of the sample are more efficiently identified and/or enriched. In particular, the fixating method enables selective lysis of maternal erythrocytes. The method comprises the steps of
a. Providing a maternal blood sample or a fraction thereof
b. Contacting the sample with a fixation solution

Also fixation solutions and uses thereof are described in the following aspects.

Another aspect of the invention relates to methods of identification and/or enrichment of fetal cells from a maternal blood sample using mRNAs preferentially expressed in fetal cells and/or proteins encoded by the mRNAs. Such methods comprises the steps of
a. Providing a maternal blood sample or a fraction thereof
b. Contacting the sample with
   i. a hybridization probe directed to a nucleic acid comprising at least 10 bp of a gene selected from the group consisting of TMEFF2, ABHD2, ACVR2B, ADAM11, AHNAK, AK000420, ALS2CL, BC089454, BC111482,CB123670, CCNA2, CD7, CPS1, CRYL1, D4ST1, DKFZp434F142, EDN1, EEF1A1, ENST00000356196, FLJ35740, FYN, GCC2, GSK3A,
      HIST1H2AJ, HLA-C, HMGA1, HMGN2, ITGA7, KCNK4, KMO, KY, LOC389286, MAD2L1, MAPK3, MYL6, NBR2, NTRK1, PF4, PGK1, PPIA, QPRT, RGPD2, RP11-78J21.1, RPL23, RPL23A, RPL26, RPL39, RPS27, SLAMF1, SYT9, T (BRACHYURY), THC2265980, THC2274391, TP73, TPM3, UBL4A, WRN, ZFYVE9, ZNF283, ZNF539, ZNF614, CD141, CD135, CD142/F3, CD146, CD33, CD68, CD144, CD62E, GAP/GJA5, ITGA5, KCNK3/TASK-1, CNTFR, JAM-1, SDC4/Syndecan 4, ACVR2B, ACVR2A, CD44v6+CD44v4/5, STX1A, TEK/Tie2, CD171, NCAM/CD56, ALPP, ALPPL2 and, MMP23A/MMP23B or
   ii. a ligand directed to an antigen encoded by a gene selected from the group consisting of TMEFF2, ABHD2, ACVR2B, ADAM11, AHNAK, AK000420, ALS2CL, BC089454, BC111482,CB123670, CCNA2, CD7, CPS1, CRYL1, D4ST1, DKFZp434F142, EDN1, EEF1A1, ENST00000356196, FLJ35740, FYN, GCC2, GSK3A, HIST1H2AJ, HLA-C,HMGA1, HMGN2, ITGA7, KCNK4, KMO, KY, LOC389286, MAD2L1, MAPK3, MYL6, NBR2, NTRK1, PF4, PGK1, PPIA, QPRT, RGPD2, RP11-78J21.1, RPL23, RPL23A, RPL26, RPL39, RPS27, SLAMF1, SYT9, T (BRACHYURY), THC2265980, THC2274391, TP73, TPM3, UBL4A, WRN, ZFYVE9, ZNF283, ZNF539, ZNF614, CD141, CD135, CD142/F3, CD146, CD33, CD68, CD144, CD62E, GAP/GJA5, ITGA5, KCNK3/TASK-1, CNTFR, JAM-1, SDC4/Syndecan 4, ACVR2B, ACVR2A, CD44v6+CD44v4/5, STX1A, TEK/Tie2, CD171, NCAM/CD56, ALPP, ALPPL2 and MMP23A/MMP23B.

After the enrichment and/or identification, the fetal cell(s) are typically subjected to a step of detection, such that a step of prediction and/or diagnosis of the fetus can be done.

### Disclosure of the invention

The present invention is based on the identification of mRNAs encoding proteins that are preferentially expressed in fetal cells of a maternal blood sample. I.e. the mRNAs are present at increased levels in the fetal cells as compared to the mRNA levels in maternal blood cells. Thus, the identified mRNAs may be used for identification of fetal cells in a maternal blood sample by detecting or quantifying the mRNA or the protein encoded by the mRNA. When the term detection is used herein, it covers both detection and quantification. In two separate embodiments however, the term detection covers either detection or quantification. Generally, the skilled man will recognize when detection also covers quantification, i.e. when it is relevant to quantify mRNA levels or the levels of the protein encoded by the mRNAs. This may e.g. be necessary for detection of a given mRNA which is expressed at a low level in maternal cells (but not absent) and where the same mRNA is expressed at e.g. 3 fold higher levels in fetal cells.

A subset of the mRNAs encode surface exposed proteins, and these proteins may be used as antigens for ligands such as aptamers or antibodies and the use of these ligands enable enrichment and/or identification of fetal cells from a maternal blood sample. Such enrichment is desirable because fetal cells obtained from maternal blood can be used for pre-natal detection and diagnosis.

Another discovery that the present inventors have made is that a step of fixating the cells of the maternal sample greatly aids identification and enrichment of fetal cells from the sample. This fixation step may be performed together with the methods of enriching and/or identifying fetal cells described herein or together with methods of enriching and/or identifying fetal cells that have been described in the prior art (e.g. US2007/0015171 described in the background section).

### Fixation of the cells of a maternal blood sample

A first aspect of the invention is based on the discovery that fixation of the cells of a maternal blood sample greatly increases stability of fetal cells in a maternal blood sample, while allowing enrichment and identification of fetal cells e.g. as further described in the second aspect of the invention. The fixation procedure can be performed on a non-enriched blood sample immediately after sampling, resulting in fixation of all cellular components in the maternal blood sample. At the same time the fixation is so mild that maternal erythrocytes can be lysed selectively in a subsequent lysis step.

Thus, a first aspect of the invention is a method comprising the steps
a. Providing a maternal blood sample or a fraction thereof
b. Contacting the sample with a fixation solution

Preferably, the maternal blood sample is contacted with the fixation solution immediately after the sample has been obtained. The term immediately as used in the present context means that the sample is not subjected to any other manipulations before being contacted with the fixation solution. Preferably, the sample is contacted with the fixation solution no more than 24 hours after the sample has been provided. More preferably, the sample is contacted with the fixation solution no more than 12 hours, such as 8 hours, 4 hours, 2 hours, 1 hour, 30 minutes, 15 minutes after the sample has been provided. Most preferably, the sample is contacted with the fixation solution no more than 1 hour after the sample has been provided.

In another preferred embodiment, the fixation solution is added to whole blood and preferably before an optional sedimentation step such as e.g. sedimentation by gravity or sedimentation by centrifugation.

Fixation is preferably done for between 1 and 60 minutes. More preferably, fixation is done for between 5 and 30 min and most preferably, fixation is done between 5 and 15 minutes such as 10 minutes.

The fixation solution preferably comprises between 2.5% and 7,5 % paraformaldehyde, more preferably between 3% and 6%, and most preferably between 4% and 5%.

In addition to paraformaldehyde, the fixation solution preferably comprises salt at a concentration between 0,05 M and 0,3 M. More preferably the concentration is between 0,1 and 0,2 M and most preferred is a concentration between 0,125 and 0,175 M. The salt is preferably LiCl, KCI, NaCl or PBS, with PBS being most preferred.

When the above mentioned concentrations of the fixation solution are used, it is preferred to add between 0,2 and 10 volumes of the fixation solution to the maternal blood sample for fixation, more preferably between 0,5 and 5 volumes is added and most preferably between 1 and 3 volumes is added . Typically 2 volumes are added. In yet another embodiment, it is preferred to add between 1/3 and 3/3 volume of fixation solution, e.g. 2/3 volume.

It will be clear to the skilled man that the various concentrations of the fixation solution and folds of dilution can be adjusted such as to give the desired final concentrations after the fixation solution has been added to maternal blood sample. Preferably, the final concentration of paraformaldehyde is between 2 and 6%, more preferably between 3 and 5 % and most preferably between 3,5% and 4,5%. A typical final concentration is 4%.

Preferably, the fixation step is followed by a step of lysis comprising:
c. Contacting the fixated sample of step a with a lysis buffer
   - wherein the lysis buffer comprises a non-ionic detergent, preferably Triton X-100. Preferred concentrations of the detergent are between 0,01 % and 0,5%, more preferably between 0,05%-0,3%, and most preferably 0,1%.

In a preferred embodiment, the lysis step is performed immediately after the fixation step.

As mentioned above, the lysis step surprisingly allows selective lysis of maternal erythrocytes.

A second aspect of the invention is the use of the fixation solution for fixing fetal cells in a maternal blood sample or a fraction thereof, as described in the first aspect. In a preferred embodiment the fixation solution is for use in the method of the fourth aspect.

A third aspect of the invention is the use of the lysis buffer for selective lysis of maternal erythrocytes in a maternal blood sample or a fraction thereof, as described in the first aspect. In a preferred embodiment, the lysis buffer is for use in the method of the fourth aspect.

### Contacting the maternal blood sample with a ligand or a probe

A fourth aspect of the present invention provides a method comprising the steps of
a. Providing a maternal blood sample or a fraction thereof
b. Contacting the sample with
   i. a hybridization probe directed to a nucleic acid comprising at least 10 bp of a gene selected from the group consisting of TMEFF2, ABHD2, ACVR2B, ADAM11, AHNAK, AK000420, ALS2CL, BC089454, BC111482,CB123670, CCNA2, CD7, CPS1, CRYL1, D4ST1, DKFZp434F142, EDN1, EEF1A1, ENST00000356196, FLJ35740, FYN, GCC2, GSK3A, HIST1H2AJ, HLA-C,HMGA1, HMGN2, ITGA7, KCNK4, KMO, KY, LOC389286, MAD2L1, MAPK3, MYL6, NBR2, NTRK1, PF4, PGK1, PPIA, QPRT, RGPD2, RP11-78J21.1, RPL23, RPL23A, RPL26, RPL39, RPS27, SLAMF1, SYT9, T (BRACHYURY), THC2265980, THC2274391, , TP73, TPM3, UBL4A, WRN, ZFYVE9, ZNF283, ZNF539, ZNF614, CD141, CD135, CD142/F3, CD146, CD33, CD68, CD144, CD62E, GAP/GJA5, ITGA5, KCNK3/TASK-1, CNTFR, JAM-1, SDC4/Syndecan 4, ACVR2B, ACVR2A, CD44v6+CD44v4/5, STX1A, TEK/Tie2, CD171, NCAM/CD56, ALPP, ALPPL2 and MMP23A/MMP23B or
   ii. a ligand directed to an antigen encoded by a gene selected from the group consisting of TMEFF2, ABHD2, ACVR2B, ADAM11, AHNAK, AK000420, ALS2CL, BC089454, BC111482,CB123670, CCNA2, CD7, CPS1, CRYL1, D4ST1, DKFZp434F142, EDN1, EEF1A1, ENST00000356196, FLJ35740, FYN, GCC2, GSK3A, HIST1H2AJ, HLA-C,HMGA1, HMGN2, ITGA7, KCNK4, KMO, KY, LOC389286, MAD2L1, MAPK3, MYL6, NBR2, NTRK1, PF4, PGK1, PPIA, QPRT, RGPD2, RP11-78J21.1, RPL23, RPL23A, RPL26, RPL39, RPS27, SLAMF1, SYT9, T (BRACHYURY), THC2265980, THC2274391, TP73, TPM3, UBL4A, WRN, ZFYVE9, ZNF283, ZNF539, ZNF614 CD141, CD135, CD142/F3, CD146, CD33, CD68, CD144, CD62E, GAP/GJA5, ITGA5, KCNK3/TASK-1, CNTFR, JAM-1, SDC4/Syndecan 4, ACVR2B, ACVR2A, CD44v6+CD44v4/5, STX1A, TEK/Tie2, CD171, NCAM/CD56, ALPP, ALPPL2 and MMP23A/MMP23B.

The gene products of the genes are identified in table 1 as are also the expression levels in fetal and maternal cells. Shown in the examples are the experiments that verify that the mRNAs of these genes are preferentially expressed in fetal cells from a maternal blood sample.

The term "a fraction thereof" is used to indicate that the maternal blood sample may be contacted directly with a ligand or a hybridization probe or that the maternal blood sample may be pre-processed such as to only comprise a fraction of the original maternal blood sample when being contacted with the ligand or hybridization probe. The maternal blood sample may e.g. be subject to concentration of its cells, a coagulation step or an enrichment step before being contacted with the ligand or hybridization probe.

### Hybridisation probes

Hybridisation probes are used as generally in the art and are typically DNA or RNA, preferably DNA. In preferred embodiments, the probes are modified with non-natural nucleotides that improve binding affinity and/or binding specificity. Preferred examples of such non-natural nucleotides are LNA (locked nucleic acids), TINA (twisted intercalating nucleic acids), PNA (peptide nucleic acid), INA (intercalating nucleic acids), morpholino and 2' O-substituted RNA monomers such as 2'O-methyl RNA monomers and 2'O-(2-methoxyethyl) RNA.

The length of the probes is preferably between 10 and 30 nucleotides, more preferably 15-25 nucleotides.

### Reporter dyes

The hybridization probes and ligands to be used according to the invention may comprise a reporter dye (also herein termed a label). Preferably, the reporter dye is selected from the group consisting of FAM™, TET™, JOE™, VIC™, SYBR® Green, 6 FAM, HEX, TET, TAMRA, JOE, ROX, Fluorescein, Cy3, Cy5, Cy5.5, Texas Red, Rhodamine, Rhodamine Green, Rhodamine Red, 6-CarboxyRhodamine 6G, Alexa Fluor, Oregon Green 488, Oregon Green 500 and Oregon Green 514.

In one embodiment, the hybridization probes also comprise a quenching dye. In a preferred embodiment, the quenching dye is selected from the group consisting of TAMRA™, Black Hole Quencher™, DABCYL, BHQ-1, BHQ-2, DDQ I, DDQ II and Eclipse Dark Quencher.

The use of reporter and quenching dye is desirable because it allows various kinds of quantifications in addition to identification.

Typically, the reporter dye and the quencher dye are located near each other in the hybridization probe, allowing light- or laser-induced fluorescence emitted by the reporter to be quenched by the quencher dye. When the oligonucleotide binds to a complementary template strand, the reporter dye and the quencher dye are separated from each other such that the quencher no longer quenches the signal from the reporter, i.e. hybridization can be detected.

Thus, in one embodiment, the hybridization probe is capable of forming a stemloop structure, wherein the quencher and reporter dye are brought into proximity in the stem. In one embodiment, the oligonucleotide is a so-called molecular beacon. The quencher and the reporter are no longer in proximity, when the molecular beacon base pairs to a template strand. Therefore the laser-induced signal from the reporter dye is no longer quenched.

Instead of using a reporter dye and a quencher dye, a so-called FRET (fluorescence resonance energy transfer) pair comprising a donor fluorophor and an acceptor fluorophor may be used. When the donor fluorophor is excited by an external light source, it emits light at a wavelength, which excites the acceptor fluorophor, which in turn emits light at a different wavelength, which can be detected and measured. The energy is only transferred from the donor to the acceptor if the donor fluorophor and acceptor fluorophor are in close proximity.

Preferred FRET pairs include BFP-YFP, CFP-YFP, GFP-DsRed, GFP-Cy3, GFP-mOrange, YFP-RFP, FAM-ROX, FAM-Cy5, FAM-Hex, FAM-TAMRA and Cy3-Cy5.

### Ligands

The ligand as used in the method of the invention is preferably an antibody, a peptide or an aptamer.

Aptamers are nucleic acid based high-affinity ligands that bind to antigens such as proteins. They are typically identified using in vitro evolution techniques such as SELEX (systematic evolution of ligands by exponential enrichment). In SELEX, iterated rounds of selection and amplification of nucleic acids from an initial library is used for identification of high-affinity aptamers. Since the initial library is very large (e.g. 10¹⁴ different sequences) and sequences may be mutated during iterated rounds, identification of high affinity aptamers can now be done on a routine basis and such methods are known to the skilled man. Preferred aptamers are less than 50 nucleotides in length.

High affinity peptides may be generated using phage display. In phage display, a library of phages displaying peptides are selected against the target and subsequently amplified in an evolution process similar to SELEX. Various systems for phage display exist and the size of the peptide may be chosen to suit particular needs. In one embodiment, the peptides to be used with the method of the invention have a size of less than 50 amino acids.

Often the library is displayed at a scaffold, e.g. an antibody scaffold. Thus, phage display may be used to identify high affinity antibodies. Other in vitro evolution techniques for antibody generation involve mRNA display, ribosome display and covalent DNA display.

Antibodies may also be generated using immunization of suitable animals such as mice, rat, goat, rabbit, horse etc.

Antibodies used for the present invention may be either monoclonal or polyclonal. Methods of generating both types of antibodies are well known to the skilled artisan. In addition to in vitro evolution methods outlined above, monoclonal antibodies are typically prepared using hybridoma technology.

### Specificity of ligands

Preferably the ligands bind specifically to fetal cells. When referring to specificity, what is meant is that the ligands have a higher binding affinity for fetal cells than for maternal cells. Binding affinity may be expressed in terms of a dissociation constant (kd) and specificity as a ratio between the kd of a given ligand for maternal cells and the kd of the same ligand for fetal cells. I.e. a ligand may have a kd of 10⁻⁵ M for maternal cells and 10⁻⁹ M for fetal cells. In this case, the specificity would be 10.000. However, since both fetal cells and maternal cells are not necessarily a homogenous population, specificity may also be expressed in terms of the fold of enrichment that can be achieved with a given ligand (as further described below).

In a preferred embodiment, the ligands are generated by the method of the fifth aspect of the invention. I.e. the specificity of the ligands has been optimized.

Preferably, the method further comprises a step of identifying fetal cells of the sample and/or a step of enriching fetal cells of the sample. In a preferred embodiment, the step of enrichment is performed before the step of identification.

After enrichment and/or identification, a step of detection and a step of prediction and/or diagnosis are often performed.

### Identification

When the method comprises a step of identification, one embodiment comprises detecting the presence of the ligand or the hybridization probe on or in the fetal cells.

Detection may be enabled by labeling the ligand or the hybridization probe with fluorescent dyes or other dyes suitable for detection. Thus, the method may e.g. be fluorescent in-situ hybridization (FISH). The probe may comprise a quencher as well as a fluorophor or a FRET pair as described above, which enables detection of hybridisation probes bound to their target sequences. Alternatively or additionally, probes binding to their targets are separated from non-binding probes by one or more washing steps.

Identification may also be done using immunostaining using a ligand such as an antibody.

Identification may be done using multicolor FISH or multicolor immunostaining. I.e. different hybridization probes with different fluorescent labels may be used simultaneously or two (or more) different antibodies with different fluorescent labels may be used simultaneously. They may both be specific for fetal cells or one may be specific for fetal cells and the other may be specific for maternal cells.

### Enrichment

In a preferred embodiment, a ligand dependent or hybridization probe dependent enrichment step is performed after the maternal sample has been contacted with the ligand or the hybridization probe. For enrichment, a ligand is preferred over a hybridization probe.

When referring to enrichment, what is meant is that the ratio of fetal cells to maternal cells of the sample is increased. The fold of enrichment is preferably more than 1000 fold, even more preferably more than 10.000 fold and most preferably more than 100.000 fold.

In another embodiment, the fold of enrichment is selected from the group consisting of more than 10 fold, more than 100 fold, more than 1000 fold, more than 10.000 fold, more than 100.000 fold and more than 1.000.000 fold.
The basis of the enrichment is the identified mRNAs preferentially expressed in fetal cells and proteins encoded by the mRNAs.

A part of the identified fetal cell specific mRNAs encode surface exposed antigens and in a preferred embodiment, the ligand is directed to a cell surface exposed antigen encoded by a gene selected from the group consisting of tomoregulin, EDN1, AHNAK, CD 105, SYT 9, KMO, HMGA1, Mouse T, ACVR2B, CD141, CD135, CD142/F3, CD146, CD33, CD68, CD144, CD62E, GAP/GJA5, ITGA5, KCNK3/TASK-1, CNTFR, JAM-1, SDC4/Syndecan 4, ACVR2B, ACVR2A, CD44v6+CD44v4/5, STX1A, TEK/Tie2, CD171, NCAM/CD56, ALPP, ALPPL2 and MMP23A/MMP23B.

The use of ligands directed to fetal cell specific surface exposed antigens enables enrichment of fetal cells from a maternal sample without permeabilizing the cells (as necessary for intracellular antigens or nucleic acids captured by hybridisation probes).

As will be clear to the person skilled in the art, additional antigen dependent enrichment steps based on ligands (or antigens) known from the prior art may be performed. Examples of such antigens known from the prior art are: CD34, Tra, Oct1, Crypto1 and SSEA1.

### Flow-based sorting

In a preferred embodiment, the enrichment is done using fluorescent activated cell sorting (FACS). Thus, the ligand is fluorescently labelled which allows FACS. FACS and suitable labels are well known to the skilled artisan and examples have been given above.

As an alternative to FACS, microfluidic device cell sorting may be used.

### Immobilization

In another preferred embodiment, enrichment is done using immobilization of the ligands. The ligands may e.g. by immobilized on beads such as magnetic beads, sepharose beads, agarose beads etc. When the ligands and cells bound thereto are immobilized, unbound cells can be washed of the beads. Such washing process may be performed in batch or on a column. After enrichment (fractionation), bound cells can be eluted using high or low salt, cleavable linkers, low or high pH, denaturing agents etc. More preferably, bound cells are eluted using competitive elution with soluble antigens or secondary ligands binding to the fetal cell specific ligands, e.g. antibodies directed to the fixed part of the ligand used for immobilization.

A preferred method of enrichment is MACS (immunomagnetic cell sorting), where the ligands are immobilized on magnetic beads. I.e. cells bound to the ligands can be separated from non-binders by selecting the particles using magnetism.

### Negative selection using antigens

Ligands that bind specifically to maternal cells may also used for enrichment. Thus, in a preferred embodiment, the method further comprises a step of contacting the sample with a maternal cell specific ligand directed to a maternal antigen. After contacting the sample with a maternal cell specific ligand, enrichment may e.g. be done using FACS, MACS, microfluidics or immobilization as described above.

Preferably, the ligand is selected from the group consisting of ligands that bind to antigens encoded by mRNAs preferentially expressed in maternal blood cells as identified by the present inventors.

As will be clear to the person skilled in the art, additional antigen dependent enrichment steps (negative selections) based on ligands (or antigens) known from the prior art may be used. Thus in one embodiment, an additional antigen dependent enrichment step is performed, where the ligand is selected from the group consisting of ligands that bind to maternal specific antigens known from the prior art such as CD45, HLA-A, HLA-B or antibodies selected from the group consisting of HLe-1, M3 and L4.

A preferred cell type marker for negative selection is CD45 also known as leukocyte common antigen. CD45 is a transmembrane protein expressed by all differentiated hematopoietic cells except erythrocytes and plasma cells. The CD45 protein exists in different forms which are all produced from a single complex gene giving rise to eight different mature mRNAs and resulting in eight different protein products. It is expressed on all leukocytes but not on other cells, and thus functions as a pan-leukocute marker including the different and diverse types of leukocytes (or white blood cells) such as neutrophils, eosinophils, basophils, lymphocyte (B and T cells), monocytes and macrophageds.

Due to the expression of CD45 on a large majority of the nucleated cells present in maternal blood a negative selection using the CD45 marker is preferred. Following depletion of CD45 positive cells, the CD45 negative cells of the sample is collected. Such depletion and collection can be performed by any suitable method known in the art.

### HLA

The human leukocyte antigens, part of the human major histocompatibility complex (MHC) is responsible for cell-surface antigen-presenting proteins and many other genes.

Two classes of the human leukocyte antigens are present, class I antigens (A, B & C) and class II antigens (DR, DP, & DQ) which have different functions. Both classes include a high number of variable alleles.
HLA genes not expressed by fetal cells may be used for depletion of maternal cells in the sample.

### Other enrichment methods

Additional enrichment methods that do not use antigen specific ligands may also be used.

A preferred additional method of enrichment is lysis of erythrocytes such as NH₄Cl mediated lysis, which allows selective lysis of erythrocytes leaving nucleated cells intact. This method is known by a person skilled in the art. For NH₄Cl mediated lysis preferably a concentration of 0.1-0.2 mM NH₄Cl is used, such as 0.14-0.18 mM NH₄Cl more preferably mM 0.15-0.17 NH₄Cl.

Also the methods of fixation and selective lysis described in the first aspect may be used for enrichment.

The sample may also be subjected to initial separation based on size or density, such as by Ficoll-Hypaque density gradient centrifugation. This results in production of a supernatant layer, which contains platelets; a mononuclear cell layer; and an agglutinated pellet which contains non-nucleated erythrocytes and granulocytes. The mononuclear layer is separated from the other layers to produce a maternal sample enriched in fetal cells.

Also physical properties of cells, such as but not exclusively charge, may be utilized for enrichment.

### Combining ligands and enrichment methods

As will be understood, the various ligands and enrichment methods may be combined. Thus, 1, 2, 3, 4, 5, 6 or more fetal cell specific ligands may be used at the same time or in succession. Likewise iterated enrichments using respectively fetal cell specific ligands and maternal specific ligands may be used.

### The sample

It is desirable to obtain as large a maternal blood sample as possible in order to increase the total number of fetal cells. However, due to practical problems the sample must be within certain limits. Accordingly, the size of the maternal blood sample is preferably in the range of 0.5 to 50 ml, such as in the range of 1 to 40 ml, such as from 5 to 35 ml or 10 to 30 ml.

The maternal blood sample provided is preferably obtained from a pregnant woman between 5 -24 or 6 -20 weeks of gestation, more preferably between 7-16, or 8-12 weeks of gestation.

### Dilution - concentration

Also, according to the invention the sample may be diluted or concentrated at anytime during the method enrichment or identification of a fetal cell (to facilitate the identification of the fetal cells and in connection with feasibility of the different method steps). The sample may be diluted at least 1.5 times, such as twice, more preferred at least three times, such as five times by adding isotonic buffers, such as saline solutions, phosphate buffered saline solutions, PBS, and/or suitable growth media, such as basal media, and tissues growth media. A method step may include dilution of a sample by addition of various components allocated for the specific method step.

For carrying out the method it may for the feasibility of the different method steps be advantageous to concentrate the sample e.g. to reduce the volume without removing any cells. The sample volume may be decreased to less than 80 %, such as 70, or 60 or 50 % of the original sample volume, or even preferable to less than 40 %, such as 25 % of the original sample volume. A concentration step may be centrifugation. The method may according to the invention comprise one or more concentration steps. Centrifugation is a preferred method for concentrating the cells. In order to avoid damages of cells a mild centrifugation is preferred, such as 300 g for 10 minutes.

### Sedimentation

The cells present in the blood sample may be concentrated by sedimentation instead of centrifugation, where the majority of cells present in the sample are allowed to sediment. The blood sample may prior to sedimentation be diluted in a suitable solution, such as 0.15 M NaCl. The sedimentation may continue until total sedimentation has occurred, such as for at least 5 hours, or preferably over night.

Preferably the sample is allowed to sediment at a temperature below room temperature, such as at a temperature of less than 15 °C, such as less than 10 °C or 8 °C or 6 °C, preferably at a temperature of 2-8 °C or around 4 °C.

A minor population of cells with a low density may not sediment and may be isolated by mild pre-fixation as described below, such as in 0.5 % paraformaldehyde followed by centrifugation.

### Fixation

In a preferred embodiment of the fourth aspect of the invention, the cells of the maternal blood sample are fixed as described in the first aspect of the invention. Thus, in a preferred embodiment, the maternal erythrocytes are selectively lysed immediately after fixation.

### Detection and diagnosis

Preferably, the method of the invention is used for prenatal detection and prediction and/or diagnosis. Thus, an identified cell may be subject to detection and prediction and/or diagnosis or a maternal blood sample enriched for fetal cells may be subjected to detection and prediction and/or diagnosis

In one embodiment, fetal proteins are made available for detection e.g. via immunoblotting, protein sequencing or mass spectrometry.

In another preferred embodiment, detection and/or diagnosis comprises a step of making fetal DNA or RNA available for detection.

Preferred detection methods are FISH (fluorescent in situ hybridization), northern blotting, southern blotting, DNA/RNA sequencing, microarray analysis and amplification. Such methods may be used to detect the presence of specific sequences that indicate a certain condition, e.g. pre-natal disease or predisposition to a certain disease. The methods may also be used to detect a chromosomal aneuploidy such as trisomy 13, trisomy 18 or trisomy 21. The detection methods can also be used to determine the gender of the fetus by detecting Y specific sequences.

In an alternative embodiment, the number of fetal cells in the sample is compared to a standard number. Increased numbers of fetal cells in the sample may indicate that the pregnancy is at risk. The number of fetal cells in the sample (as well as in a control sample) can be estimated using e.g. FACS.

### Identification of specific ligands

A fifth aspect of the invention is a method of identifying a fetal cell specific ligand comprising the steps:
a) Providing a library of fetal cell specific ligand candidates
b) Providing a pool of maternal cells
c) Contacting the library of step a with the maternal cells of step b
d) Selecting ligands that do not bind to the maternal cells to generate a library depleted for ligands that bind maternal cells

In a preferred embodiment, the method further comprises the steps of
e) Contacting the library of step a or the library of step d with a fetal cell
f) Selecting ligands that bind to the fetal cell to generate a library that is enriched in ligands that bind to fetal cells, but not maternal cells

It should be clear that one cell suffices for selection of the ligands of step f, but that more fetal cells may obviously be used.

Steps b-f may be carried out by the steps of
g) Providing a maternal blood sample
h) Contacting the library with a maternal blood sample
i) Selecting ligands that bind to the fetal cells by removing individual fetal cells, which have been identified by FISH-demonstration of a Y chromosome, and collecting the ligands solely from these cells.

The maternal blood sample may have been enriched for fetal cells.

In a preferred embodiment, the method further comprises:
j) multiplying/amplifying the selected ligands such as to prepare an amplified library for additional selections against fetal cells and/ or against maternal cells.

As will be clear, multiple rounds of selection and amplification may be performed to identify the very best ligands.

The library of fetal cell specific ligand candidates may be a library of antibodies or peptides displayed on phages (phage display), mRNA (ribosome display or mRNA display) or on DNA (covalent display or plasmid panning). The library may also be a library of DNA or RNA oligonucleotides for the identification of aptamers.

The term "candidates" is used to imply that the compounds of the library do not necessarily bind to fetal cells. They are to be tested for binding for the identification of fetal cell specific ligands.

In one embodiment, the library of fetal cell specific ligand candidates is a fully random library. In such case, the library may first be iteratively selected against fetal cells and amplified, before counter selection (negative selection) against maternal cells is performed.

In another embodiment, the library of fetal cell specific ligand candidates is based upon known ligands of fetal cells. Such library may e.g. be created by displaying an antibody that binds to fetal cells on a phage and mutagenesis of the gene encoding the antibody to create a library. In such case, mutagenesis may improve specificity while retaining or even improving affinity for fetal cells.

In one embodiment, the ligand binds to an antigen encoded by a gene selected from the group consisting of tomoregulin, EDN1, AHNAK, CD 105, SYT 9, KMO, HMGA1, Mouse T, ACVR2B, CD141, CD135, CD142/F3, CD146, CD33, CD68, CD144, CD62E, GAP/GJA5, ITGA5, KCNK3/TASK-1, CNTFR, JAM-1, SDC4/Syndecan 4, ACVR2B, ACVR2A, CD44v6+CD44v4/5, STX1A, TEK/Tie2, CD171, NCAM/CD56, ALPP, ALPPL2 and MMP23A/MMP23B. Thus affinity and/or specificity of the ligand are optimized using the method outlined above.

### Fetal cell specific ligands and hybridization probes

A sixth aspect of the invention is
i. a ligand that binds an antigen encoded by a gene selected from the group consisting of TMEFF2, ABHD2, ACVR2B, ADAM11, AHNAK, AK000420, ALS2CL, BC089454, BC111482,CB123670, CCNA2, CD7, CPS1, CRYL1, D4ST1, DKFZp434F142, EDN1, EEF1A1, ENST00000356196, FLJ35740, FYN, GCC2, GSK3A, HIST1H2AJ, HLA-C,HMGA1, HMGN2, ITGA7, KCNK4, KMO, KY, LOC389286, MAD2L1, MAPK3, MYL6, NBR2, NTRK1, PF4, PGK1, PPIA, QPRT, RGPD2, RP11-78J21.1, RPL23, RPL23A, RPL26, RPL39, RPS27, SLAMF1, SYT9, T (BRACHYURY), THC2265980, THC2274391, TP73, TPM3, UBL4A, WRN, ZFYVE9, ZNF283, ZNF539, ZNF614, CD141,
   CD135, CD142/F3, CD146, CD33, CD68, CD144, CD62E, GAP/GJA5, ITGA5, KCNK3/TASK-1, CNTFR, JAM-1, SDC4/Syndecan 4, ACVR2B, ACVR2A, CD44v6+CD44v4/5, STX1A, TEK/Tie2, CD171, NCAM/CD56, ALPP, ALPPL2 and MMP23A/MMP23B
ii. or a hybridization probe directed to a nucleic acid comprising at least 10 bp of a gene selected from the group consisting of TMEFF2, ABHD2, ACVR2B, ADAM11, AHNAK, AK000420, ALS2CL, BC089454, BC111482,CB123670, CCNA2, CD7, CPS1, CRYL1, D4ST1, DKFZp434F142, EDN1, EEF1A1, ENST00000356196, FLJ35740, FYN, GCC2, GSK3A, HIST1H2AJ, HLA-C,HMGA1, HMGN2, ITGA7, KCNK4, KMO, KY, LOC389286, MAD2L1, MAPK3, MYL6, NBR2, NTRK1, PF4, PGK1, PPIA, QPRT, RGPD2, RP11-78J21.1, RPL23, RPL23A, RPL26, RPL39, RPS27, SLAMF1, SYT9, T (BRACHYURY), THC2265980, THC2274391, TP73, TPM3, UBL4A, WRN, ZFYVE9, ZNF283, ZNF539, ZNF614, CD141, CD135, CD142/F3, CD146, CD33, CD68, CD144, CD62E, GAP/GJA5, ITGA5, KCNK3/TASK-1, CNTFR, JAM-1, SDC4/Syndecan 4, ACVR2B, ACVR2A, CD44v6+CD44v4/5, STX1A, TEK/Tie2, CD171, NCAM/CD56, ALPP, ALPPL2 and MMP23A/MMP23B.

More preferably, the ligand is a ligand that binds an antigen encoded by a gene selected from the group consisting of tomoregulin, EDN1, AHNAK, CD 105, SYT 9, KMO, HMGA1, Mouse T, ACVR2B, CD141, CD135, CD142/F3, CD146, CD33, CD68, CD144, CD62E, GAP/GJA5, ITGA5, KCNK3/TASK-1, CNTFR, JAM-1, SDC4/Syndecan 4, ACVR2B, ACVR2A, CD44v6+CD44v4/5, STX1A, TEK/Tie2, CD171, NCAM/CD56, ALPP, ALPPL2 MMP23A/MMP23B i.e. surface exposed antigens. Preferably the kd of the ligand for the chosen antigen is less than 10⁻⁸ M, 10⁻⁹ and 10⁻¹⁰ M. The kd of the ligand for any antigens present on maternal blood cells is preferably at least 100 fold higher and even more preferably more than 1000 or 10.000 fold higher.

In one embodiment, the ligand is characteristic in that it enables 90% correct selection of cells in a test sample comprising 99,9% maternal cells and 0,1 % fetal cells. I.e. when referring to 90 % correct identification, what is meant herein is that when performing the selection with the test sample and with the ligand, 90 fetal cells will be collected for each 10 maternal cells and likewise for better/worse correctness. A preferred selection method is MACS. More preferred is a ligand that enables 95% correct selection, 98% correct selection or even more preferred 99% correct cell selection. Since a maternal blood sample has a very low abundance of fetal cells, it is even more preferred that the ligand enables 99,9%, 99,99% or 99,999% correct cell selection from a test sample as described above.

Preferably, the ligands of the sixth aspects are aptamers, peptides or antibodies as described in the fourth and fifth aspect. Most preferred are antibodies.

The ligands are preferably identified using the method of the fifth aspect such as to have an improved specificity.

A seventh aspect of the invention is use of the ligands or hybridization probes of the sixth aspect for enriching a maternal blood sample for fetal cells or for identifying fetal cells in a maternal blood sample. Preferably use of the ligands or the hybridization probes is as described in the fourth aspect of the invention.

An eight aspect of the invention is a kit comprising a ligand or a hybridization probe as described in the sixth aspect of the invention and instructions for use.

Preferably, the kit comprises a first ligand for enrichment and a second ligand and/or a hybridization probe for identification.

In a preferred embodiment, the kit also comprises a fixation buffer and a lysis buffer as described in the first aspect of the invention.

### Examples

Example 1 is written in present tense because it outlines methodic steps to be performed when enriching and/or identifying fetal cells from a maternal blood sample using the methods of the invention. cDNA synthesis and microarrays is typically not necessary for enrichment and/identification, but may be used in some situations. It should be clear that the steps described in example 1 have actually been carried out for the identification of mRNAs preferentially expressed in fetal cells from a maternal blood sample.

### Example 1

Identification of mRNAs preferentially expressed in fetal cells of a maternal blood sample.

### Fixation, lysis and permeabilisation of maternal blood.

Peripheral blood samples of 10 to 30 ml are obtained from pregnant women 11 to 14 week's gestational age. Blood samples are drawn before an invasive procedure and after informed consent. All blood samples are collected in heparinized tubes and processed immediately after they were collected.

In addition to the heparin blood, 5 ml of blood is drawn in EDTA tubes. This blood is used for fetal gender analysis. The gender of the fetus is established by analyzing the free fetal DNA.

For each sample 3 ml of whole blood is aliquoted into pre-coated 50 ml centrifugation tubes (3 to 10 50 ml tubes per sample) using pre-coated pipettes (pre-coating buffer is 2% BSA in PBS w/o Ca2+, Mg2+). Two ml of 10% formaldehyde in PBS is added to each tube using pre-coated pipettes. After careful mixing the blood cells are fixed for 10 minutes at room temperature.

After fixation 30 ml of 0, 12% Triton X-100 in PBS (w/o Ca2+, Mg2+) is added to each tube. The tubes are inverted 3 times, and the red blood cells are lysed for 45 minutes at room temperature. Following lysis, 15 ml cold (4°C) 2% BSA in PBS (w/o Ca2+, Mg2+) is added to each tube. After mixing by inverting the tubes 2 times, unlysed cells are pelleted by centrifugation at 500 g for 15 minutes at 40 C. After removing the supernatant, the cells are resuspended in 10 ml of 40 C cold PBS w/o Ca2+, Mg2+, and the samples are stored at 4° C overnight.

Only blood samples from women pregnant with a male fetus is analysed further. These samples are permeabilised by adding 10 ml of cold methanol (- 20° C) to the 10 ml cell suspension store overnight, and the cells are permeabilised for 10 minutes at 4° C. After centrifugation at 500 g for 10 minutes, the cell pellets are pooled into one tube using pre-coated pipettes. The empty tubes are rinsed with 1 ml of PBS, 0,5% BSA, 2 mM EDTA. The pooled cells are then transferred to a 15 ml tube and centrifuged at 500 g for 10 minutes and re-suspended in what corresponds to 40 ul of PBS containing 0,5% BSA and 2 mM EDTA per 1 ml of whole blood, and the cells are smeared onto polylysine coated slides (4 µl each). A total of 60 slides are made from each male blood sample. After air-drying the slides are sealed individually in airtight plastic bags and stored at -20° C until further analysis.

Identification of male fetal cells by reverse color FISH and automated scanning. Slides are recovered from the freezer and the airtight plastic bags are removed. Before hybridization, slides are rinsed in phosphate buffered saline (PBS) and for 5 minutes before they are dehydrated 3 minutes each in 60%, 80% and 99,9% ethanol and air-dried.

Chromosome-specific repeat probes, DXZ1 labeled with spectrum green and DYZ1 labeled with spectrum orange (Abbott Molecular) are used for the first hybridization. Hybridization mixture containing both probes is prepared by mixing 1 part of the X-probe, 1 part of the Y-probe, 1 part of distilled water, and 7 parts of hybridization buffer (Vysis). For whole slide FISH, 28 µl of the hybridization mixture is added and covered by a 22 x 50mm cover slips. Cover slips are sealed with rubber cement and the DNA's are denatured on a hot plate at 83.5 °C for 7 minutes and hybridized overnight in a humidified atmosphere at 42 °C.

The next day hybridized slides are washed for 2 minutes in 0.4 x SSC/0,3% Tween 20 at 73 °C and for 1 minute in 2 x SSC/0,1% Tween 20 at room temperature. The slides are then mounted in Vectashield with 1,5 µg/ml DAPI as counter stain.

Cells containing a red signal located in a DAPI stained nucleus is identified by automatic scanning using two different types of scanners. The MDS (version 5.8.0) slide scanning system originally developed by Applied Imaging, and the MetaCyte scanning system developed by MetaSystems. With the MDS scanning system slides are scanned at 20x magnification using scan function 5. With MetaCyte slides are scanned at 10x magnification using a classifier optimized in-house for detecting true Spectrum Orange FISH signals. After scanning, cells identified by the scanner are inspected visually by automatic relocation. Cells that have one red signal but two green X-signals are discarded, while cells that have one red signal and one green X-signal or a split green X-signal are classified as candidate male fetal cells.

The true fetal origin of the candidate fetal cells is analyzed by re-hybridization of candidate cells with the same X and Y probes in reverse colors. First, the cover slips are removed by incubating the slides in 4xSSC/0,1% Tween 20 for 10 minutes. The slides are then washed in 2xSSC for 5 minutes, dehydrated through 60%, 80%, and 99,9% ethanol and air-dried. Hybridization mixture containing 1 part of the chromosome-specific repeat probes, DXZ1 labeled with spectrum red, 1 part of the chromosome-specific repeat probe DYZ1 labeled with spectrum green, 1 part distilled water, and 7 parts hybridization buffer is used for re-hybridization. For whole slide FISH, 28 µl of the hybridization mixture is added and covered by a 22 x 50 mm cover slips. For selective FISH, 2.5 µl hybridization mixture is applied at positions on the slides where candidate fetal cells had been found and the mixture is spread by covering with a 10mm circular cover slip. Cover slips are then sealed with rubber cement, and DNA's is denatured on a hot plate at 83.5 °C for 7 minutes and hybridized overnight in a humidified atmosphere at 42 °C.

After re-hybridization slides are washed for 2 minutes in 0.4 x SSC/0,3 % Tween 20 at 73 °C and for 1 minute in 2 x SSC/0,1% Tween 20 at room temperature. The slides are then mounted in Vectashield containing DAPI.

Re-hybridized slides are placed in the scanning microscope and re-hybridized candidate fetal cells are relocated and FISH signals are analyzed visually. Re-hybridized candidate fetal cells where the red signal remains red and the green signal has switched to red are classified as false fetal cells (< 5%), whereas re-hybridized candidate fetal cells where the red signal has switched to green and the green signal to red are classified as true fetal cells (> 95%).

PALM

When 23 fetal cells has been detected by re-hybridisation, the exact positions (coordinates) of the individual fetal cells on the microscope slides are determined by using an Englandfinder, so the fetal cells can be re-found on other microscopes.

The fetal cells are shot off the microscope slides by using the Laser Microdissection and Pressure Catapulting (LMPC) technology on a PALM MicroBeam microscope system from Carl Zeiss. The microscope slides containing fetal cells are transported to Carl Zeiss MicroImaging GmbH, (located in Munich, Germany), where the fetal cells are re-found by using their specific coordinates on the Englandfinder. The coverslips are carefully removed after soaking the slides in PBS for 10-15 minutes. The slides are then rinsed for 10 minutes in PBS before they are dehydrated 3 minutes each in 60%, 80% and 99,9% ethanol and air-dried. The fetal cells are then shot off the slide into an adhesive cap of a PCR sized tube by laser catapulting using a PALM MicroBeam microscope. All 23 fetal cells are collected in the same cap. In another cap maternal control cells are collected: From each slide containing a fetal cell, 100 surrounding maternal cells are collected into the control cap, giving a total number of 2300 maternal control cells. The two tubes containing fetal cells and control cells in their caps, respectively, are sent to Miltenyi Biotec GmbH, Bergisch Gladbach, Germany, for further analysis.

The RNA of the fetal sample and control sample is amplified by Miltenyi Biotec by their SuperAmp Service as described on their homepage. The amplified cDNA is used for making two different types of Microarrays: Agilent Whole Genome Microarray and the PIQOR™ Stem Cell Microarray, as described on Miltenyi Biotec's homepage.

From the microarray results, 61 genes (listed in table 1) are selected first as likely candidates for fetal cell markers. They are selected on the basis of three different criterions: 41 genes are selected because they have 2 or more different probes on the array showing strong signals in the fetal Agilent whole genome array and no or very weak signals in the maternal Agilent whole genome array. 10 genes are selected because of very strong signals on the fetal Agilent whole genome array. The last 10 genes are selected because they in both Agilent whole genome arrays and the PIQOR™ Stem Cell Microarray show signals on the fetal array (Agilent) / channel (PIQOR™ Stem Cell Microarray), but not in the maternal array/channel. The 61 genes are studied thoroughly in the literature and on the basis of this, 9 genes are selected as cell surface exposed fetal cell marker candidates: EDN1, AHNAK, tomoregulin, CD 105, SYT 9, KMO, HMGA1, Mouse T, ACVR2B.

**Table 1:**

| **Gene Name** | **Systematic Name** | **Description** | **Maternal signal** | **Fetal signal** |
|---|---|---|---|---|
| ZNF614 | NM_025040 | Homo sapiens zinc finger protein 614 (ZNF614), mRNA [NM_025040] | 2,50 | 52,61 |
| ZNF539 | NM_203282 | Homo sapiens zinc finger protein 539 (ZNF539), mRNA [NM_203282] | 1,66 | 20,81 |
| ZNF283 | AK098175 | Homo sapiens cDNA FLJ40856 fis, clone TRACH2016498, moderately similar to ZINC FINGER PROTEIN 184. [AK098175] | 1,21 | 773,52 |
| ZFYVE9 | NM_004799 | Homo sapiens zinc finger, FYVE domain containing 9 (ZFYVE9), transcript variant 3, mRNA [NM_004799] | 1,86 | 785,02 |
| WRN | NM_000553 | Homo sapiens Werner syndrome (WRN), mRNA [NM_000553] | 0,86 | 13,39 |
| UBL4A | NM_014235 | Homo sapiens ubiquitin-like 4A (UBL4A), mRNA [NM_014235] | 0,87 | 80,27 |
| TPM3 | NM_153649 | Homo sapiens tropomyosin 3 (TPM3), transcript variant 2, mRNA [NM_153649] | 1,03 | 25,86 |
| TP73 | NM_005427 | Homo sapiens tumor protein p73 (TP73), mRNA [NM_005427] | 0,89 | 14,53 |
| TMEFF2 | AB004064 | Homo sapiens mRNA for tomoregulin, complete cds. [AB004064] | 2,73 | 899,23 |
| THC2274391 | THC2274391 | RIFK_HUMAN (Q969G6) Riboflavin kinase (ATP:riboflavin 5'-phosphotransferase) (Flavokinase) , complete [THC2274391] | 0,87 | 893,63 |
| THC2265980 | THC2265980 | T97996 ye56c04.s1 Soares fetal liver spleen 1NFLS Homo sapiens cDNA clone IMAGE:121734 3' similar to SP: RL2B_RAT P29316 60S RIBOSOMAL PROTEIN ;, mRNA sequence [T97996] | 0,92 | 62,58 |
| T (BRACHYURY) | NM_003181 | Homo sapiens T, brachyury homolog (mouse) (T), mRNA [NM_003181] | 5,38 | 44,45 |
| SYT9 | NM_175733 | Homo sapiens synaptotagmin IX (SYT9), mRNA [NM_175733] | 0,89 | 1567,5 0 |
| SLAMF1 | NM_003037 | Homo sapiens signaling lymphocytic activation molecule family member 1 (SLAMF1), mRNA [NM_003037] | 1,05 | 62,58 |
| RPS27 | NM_001030 | Homo sapiens ribosomal protein S27 (metallopanstimulin 1) (RPS27), mRNA [NM_001030] | 0,90 | 639,19 |
| RPL39 | NM_001000 | Homo sapiens ribosomal protein L39 (RPL39), mRNA [NM_001000] | 0,87 | 869,05 |
| RPL26 | NM_000987 | Homo sapiens ribosomal protein L26 (RPL26), mRNA [NM_000987] | 0,92 | 313,51 |
| RPL23A | NM_000984 | Homo sapiens ribosomal protein L23a (RPL23A), mRNA [NM_000984] | 0,91 | 25,80 |
| RPL23 | NM_000978 | Homo sapiens ribosomal protein L23 (RPL23), mRNA [NM_000978] | 2,28 | 943,60 |
| RP11-78J21.1 | NM_00101172 4 | Homo sapiens heterogeneous nuclear ribonucleoprotein A1-like (LOC144983), transcript variant 1, mRNA [NM_001011724] | 0,89 | 52,05 |
| RGPD2 | NM_00102445 7 | Homo sapiens RANBP2-like and GRIP domain containing 2 (RGPD2), mRNA [NM_001024457] | 2,19 | 715,07 |
| QPRT | NM_014298 | Homo sapiens quinolinate phosphoribosyltransferase (nicotinate-nucleotide pyrophosphorylase (carboxylating)) (QPRT), mRNA [NM_014298] | 1,95 | 214,40 |
| PPIA | NM_203430 | Homo sapiens peptidylprolyl isomerase A (cyclophilin A) (PPIA), transcript variant 2, mRNA [NM_203430] | 0,88 | 230,29 |
| PGK1 | NM_000291 | Homo sapiens phosphoglycerate kinase 1 (PGK1), mRNA [NM_000291] | 0,93 | 22,71 |
| PF4 | NM_002619 | Homo sapiens platelet factor 4 (chemokine (C-X-C motif) ligand 4) (PF4), mRNA [NM_002619] | 0,97 | 8,48 |
| NTRK1 | NM_002529 | Homo sapiens neurotrophic tyrosine kinase, receptor, type 1 (NTRK1), transcript variant 2, mRNA [NM_002529] | 2,77 | 10,84 |
| NBR2 | NM_005821 | Homo sapiens neighbor of BRCA1 gene 2 (NBR2), mRNA [NM_005821] | 0,99 | 247,30 |
| MYL6 | NM_079423 | Homo sapiens myosin, light polypeptide 6, alkali, smooth muscle and non-muscle (MYL6), transcript variant 2, mRNA [NM_079423] | 2,19 | 4314,1 5 |
| MAPK3 | NM_002746 | Homo sapiens mitogen-activated protein kinase 3 (MAPK3), transcript variant 1, mRNA [NM_002746] | 1,22 | 10,99 |
| MAD2L1 | NM_002358 | Homo sapiens MAD2 mitotic arrest deficient-like 1 (yeast) (MAD2L1), mRNA [NM_002358] | 0,96 | 9,16 |
| LOC389286 | NM_00101802 2 | Homo sapiens similar to FKSG62 (LOC389286), mRNA [NM_001018022] | 0,89 | 378,70 |
| KY | NM_178554 | Homo sapiens kyphoscoliosis peptidase (KY), mRNA [NM_178554] | 0,95 | 868,36 |
| KMO | NM_003679 | Homo sapiens kynurenine 3-monooxygenase (kynurenine 3-hydroxylase) (KMO), mRNA [NM_003679] | 0,88 | 163,00 |
| KCNK4 | NM_033310 | Homo sapiens potassium channel, subfamily K, member 4 (KCNK4), mRNA [NM_033310] | 12,21 | 204,06 |
| ITGA7 | NM_002206 | Homo sapiens integrin, alpha 7 (ITGA7), mRNA [NM_002206] | 0,92 | 10,02 |
| HMGN2 | NM_005517 | Homo sapiens high-mobility group nucleosomal binding domain 2 (HMGN2), mRNA | 2,16 | 22,05 |
| | | [NM_005517] | | |
| HMGA1 | NM_002131 | Homo sapiens high mobility group AT-hook 1 (HMGA1), transcript variant 2, mRNA [NM_002131] | 1,81 | 74,66 |
| HLA-C | BC002463 | Homo sapiens major histocompatibility complex, class I, C, mRNA (cDNA clone MGC:2285 IMAGE:3345005), complete cds. [BC002463] | 0,98 | 40,27 |
| HIST1H2AJ | NM_021066 | Homo sapiens histone 1, H2aj (HIST1H2AJ), mRNA [NM_021066] | 2,97 | 49,45 |
| GSK3A | NM_019884 | Homo sapiens glycogen synthase kinase 3 alpha (GSK3A), mRNA [NM_019884] | 2,81 | 1780,9 7 |
| GCC2 | NM_181453 | Homo sapiens GRIP and coiled-coil domain containing 2 (GCC2), transcript variant 1, mRNA [NM_181453] | 2,80 | 831,61 |
| FYN | NM_002037 | Homo sapiens FYN oncogene related to SRC, FGR, YES (FYN), transcript variant 1, mRNA [NM_002037] | 0,90 | 28,36 |
| FLJ35740 | NM_147195 | Homo sapiens FLJ35740 protein (FLJ35740), mRNA [NM_147195] | 0,99 | 15,22 |
| ENST00000356196 | ENST00000356196 | PREDICTED: Homo sapiens similar to 60S ribosomal protein L26 (Silica-induced gene 20 protein) (SIG-20) (LOC400055), mRNA [XM_374987] | 2,23 | 940,84 |
| EEF1A1 | NM_001402 | Homo sapiens eukaryotic translation elongation factor 1 alpha 1 (EEF1A1), mRNA [NM_001402] | 1,29 | 160,32 |
| EDN1 | NM_001955 | Homo sapiens endothelin 1 (EDN1), mRNA [NM_001955] | 0,89 | 404,58 |
| DKFZp434F142 | AL136837 | Homo sapiens mRNA; cDNA DKFZp434F142 (from clone DKFZp434F142). [AL136837] | 2,53 | 310,56 |
| D4ST1 | NM_130468 | Homo sapiens dermatan 4 sulfotransferase 1 (D4ST1), mRNA [NM_130468] | 1,79 | 25,13 |
| CRYL1 | NM_015974 | Homo sapiens crystallin, lambda 1 (CRYL1), mRNA [NM_015974] | 1,93 | 15,35 |
| CPS1 | NM_001875 | Homo sapiens carbamoylphosphate synthetase 1, mitochondrial (CPS1), mRNA [NM_001875] | 0,91 | 35,55 |
| CD7 | NM_006137 | Homo sapiens CD7 molecule (CD7), mRNA [NM_006137] | 20,39 | 145,67 |
| CCNA2 | NM_001237 | Homo sapiens cyclin A2 (CCNA2), mRNA [NM_001237] | 0,88 | 52,42 |
| CB123670 | CB123670 | K-EST0172083 L12JSHC0 Homo sapiens cDNA clone L12JSHC0-2-D12 5', mRNA sequence [CB123670] | 1,53 | 812,97 |
| BC111482 | BC111482 | Homo sapiens cDNA clone IMAGE:5743861, with apparent retained intron. [BC111482] | 0,88 | 51,28 |
| BC089454 | BC089454 | Homo sapiens cDNA clone MGC: 105145 IMAGE:30563285, complete cds. [BC089454] | 0,84 | 51,55 |
| ALS2CL | NM_147129 | Homo sapiens ALS2 C-terminal like (ALS2CL), transcript variant 1, mRNA [NM_147129] | 2,96 | 14,83 |
| AK000420 | AK000420 | Homo sapiens cDNA FLJ20413 fis, clone KAT02170. [AK000420] | 0,91 | 113,52 |
| AHNAK | NM_001620 | Homo sapiens AHNAK nucleoprotein (desmoyokin) (AHNAK), transcript variant 1, mRNA [NM_001620] | 2,53 | 1672,1 3 |
| ADAM11 | NM_002390 | Homo sapiens ADAM metallopeptidase domain 11 (ADAM11), mRNA [NM_002390] | 0,99 | 21,49 |
| ACVR2B | NM_001106 | Homo sapiens activin A receptor, type IIB (ACVR2B), mRNA [NM_001106] | 5,13 | 39,23 |
| ABHD2 | NM_007011 | Homo sapiens abhydrolase domain containing 2 (ABHD2), transcript variant 1, mRNA [NM_007011] | 0,96 | 21,99 |

### Example 2

### Identification of fetal cells from a maternal blood sample using ligands.

### Depletion of maternal CD45 and GPA positive cells.

Maternal blood samples used for the identification of fetal cells using fetal cell specific ligands are processed as described in Example 1. After the final centrifugation at 500g for 10 minutes the supernatant is discarded, and the cell pellet is re-suspended in 80 µl per 107 cells cold PBS with 0,5% BSA and 2 mM EDTA using a pre-coated micro tip. 20 µl CD45 microbeads and 40 µl GPA microbeads is added. After mixing using a pre-coated micro tip the cells are incubated for 15 minutes at 40C. Cells are washed by adding 5 ml of cold PBS with 0,5% BSA and 2 mM EDTA and centrifuged at 500g for 10 minutes. The supernatant is removed, and the cell pellet is re-suspended in 2 ml PBS with 0,5% BSA and 2 mM EDTA using a pre-coated pipette tip. The cell suspension is loaded onto a pre-washed LD column and the flow through is collected in a fresh pre-coated tube. When the cell suspension has run through, the column is washed 3 times with 1 ml of cold PBS with 0,5% BSA and 2 mM EDTA. The tube containing the flow-through is then centrifuged at 500g for 10 minutes. The supernatant is discarded and the cell pellet is re-suspended in 400 - 600 µl (if 24 ml of blood) PBS with 0,5% BSA and 2 mM EDTA using pre-coated pipette tips and the cells are placed on polylysine coated slides and the slides are air-dried over-night. A 24 ml blood sample normally gives 10 to 15 slides with a cell smear area of 15 x 15 mm per slide.

### Identification of fetal cells.

Fetal cells are identified using an antibody against the product of one of the genes preferentially expressed in fetal cells such as for example synaptotagmin IX which is the product of SYT9.

After air drying, slides are re-hydrated in 4xSSC for 5 minutes, then pre-incubated for 30 minutes at room temperature with 100 µl of blocking buffer consisting of 4xSSC containing 1% BSA and 0,5% blocking reagent (Roche). Slides are then incubated for 30 minutes at RT with 100 µl biotinylated primary antibody solution diluted in blocking buffer according to manufactures instructions. After antibody incubation, slides are washed three times for 5 minutes in 4xSSC. To detect antibody bound to foetal cells, the slides are incubated with 100 µl streptavidin conjugated to FITC diluted 1:100 in blocking buffer for 30 minutes at RT. After washing two times for 5 minutes each in 4xSSC and one time for 5 minutes in 2xSSC, slides were mounted in Vectashield containing 1,5 µg 4,6-diamidino-2-phenylindole (DAPI) to counterstain the nuclei.

Antibody stained fetal cells are identified by automatic scanning using the Metafer program developed by Metasystems. The slides are scanned at 10x magnification using an in-house developed classifier optimized for detecting synaptotagmin stained cells. The true fetal origin of antibody stained cells can be confirmed by XY FISH as described in Example 1.

### Example 3

### Enrichment of fetal cells from a maternal blood sample

### Antibody labeling.

The maternal blood samples used for enrichment of fetal cells using a fetal cell specific ligand is processed as described in Example 1. After the final centrifugation at 500 g for 10 minutes, the supernatant is discarded, and the cell pellet is re-suspended in 95 µl of cold PBS with 0,5% BSA and 2 mM EDTA per 10 ⁷ cells. 5 µl per 10⁷ cells of biotinylated TMEFF antibody is added using pre-coated pipettes tips. After mixing, the cells are incubated for 30 to 60 minutes at either 40C or room temperature. After incubation, the cells are washed by adding 5 ml of cold PBS with 0,5% BSA and 2 mM EDTA. After centrifugation for 10 minutes at 500 g, the supernatant is discarded and the cell pellet is re-suspended in 80 µl cold PBS with 0,5% BSA and 2 mM EDTA per 10⁷ cells. 20 µl of anti-biotin microbeads per 10⁷ cells is added using a pre-coated pipette tip. After mixing the cells are incubated for 30 minutes at 40C. The cells are again washed by adding 5 ml of cold PBS with 0,5% BSA and 2 mM EDTA. After centrifugation for 10 minutes at 500 g, the supernatant is discarded, and the cell pellet is re suspended in 5 ml of cold PBS with 0,5% BSA and 2 mM EDTA.

### Positive selection using MACS

One pre-washed LD column and one pre-washed MS column is placed on the magnets, stacked. The suspension of labeled cells is applied to the upper LD column. When the cells have run through the LD column, it is washed twice with 2 ml and cold PBS with 0,5% BSA and 2 mM EDTA. The LD column is removed from the magnet and placed on a fresh pre-coated 15 ml centrifugation tube. The cells are eluted by applying 2 times 5 ml of cold PBS with 0,5% BSA and 2 mM EDTA. The first 5 ml of buffer run through the column without applying the plunger. The second 5 ml of buffer runs through the column by applying the plunger. The MS column is then removed from the magnet and placed on the collection tube. The cells are eluted the same way as for the LD column using 2 times 1 ml of cold buffer instead of 2 x 5 ml. The collection tube is centrifuged for 10 minutes at 500 g. The supernatant is discarded and the cell pellet is re-suspended in cold PBS with 0,5% BSA and 2 mM EDTA. The cell suspension is then placed on a polylysine coated slide, and the slide is air-dried before further analysis.

### Detection of male fetal cells

Male fetal cells isolated using the TMEFF antibody are identified by XY FISH followed by automatic scanning and manual validation as described in Example 1. The fetal origin of fetal candidate cells is confirmed by reverse color FISH as described in Example 1.

### Example 4

### Chromosome analysis of fetal cells from maternal blood

Analysis for Down's syndrome in fetal cells from maternal blood is done by FISH using the LSI 21 from Abbott Molecular. Before FISH, the coverslip is washed by 10 minutes incubation in 2xSSC. The slide is then rinsed in PBS, and dehydrated in increasing concentrations of ethanol. After air-drying, 10 µl of the probe mixture is applied at the position on the slide where the fetal cells are located and the mixture is spread by covering the area with a 22 x 22 mm cover slip. The cover slip is then sealed with rubber cement, and the DNA's are denatured on a hot plate for 7 minutes at 83,5 °C, and hybridized over night in a humidified atmosphere at 37°C. The next day, hybridized slides are washed at 73°C for 2 minutes in 0,4 x SSC with 0,3% Tween 20 followed by 1 minute in 2 x SSC with 0,1% Tween 20 at room temperature. The slides are mounted in Vectashield containing 1,5 µg/ml DAPI as a counterstain. Numbers of chromosome 21 FISH signals present in fetal cells is counted in the microscope by relocating to the specific cells using the original scan file.

### Example 5

Identification of mRNAs preferentially expressed in fetal cells of a maternal blood sample, enriched using CD105 positive selection.

Peripheral blood samples from pregnant women are fixed, the red blood cells lysed, and white blood cells permeabilized as described in Example 1.

To 500 µl cell suspension 130 µl of CD105 microbeads (Miltenyi) were added and the cell suspension was incubated for 60 minutes at 40C. The cells were then washed by adding 6 ml of cold MACS buffer followed by a centrifugation for 10 minutes at 500 g. The supernatant was removed and the cells re-suspended in 2 ml of cold MACS buffer.

The CD105 labeled cell suspension was applied to a pre-washed MS column (Miltenyi) already in place on the magnet and stacked on top of a pre-washed MS column (Miltenyi). When the cells had run through the LD column, it was washed twice with 2 ml of cold MACS buffer. The MS column was washed with 1 ml of cold MACS buffer. The LD column was then removed from the magnet, placed on a pre-coated 15 ml tube, and the cells were eluted by applying 2 times 5 ml of cold MACS buffer. The first 5 ml of buffer ran through the column without applying a plunger. The second 5 ml of buffer was forced through the column by applying a plunger. The MS column was then removed from the magnet and placed on the collection tube. The cells were eluted the same way as for the LD column using 2 times 1 ml of cold MACS buffer instead of 2 times 5 ml of buffer. The collection tube was centrifuged at 500 g for 10 minutes. The supernatant was discarded and the cell pellet was re-suspended in cold MACS buffer. The cell suspension was then placed on poly-lysine coated slides, and the slides were air-dried (overnight) before further analysis.

Male fetal cells were identified by X- and Y-chromosome specific FISH and automated scanning. Before hybridization, slides were rinsed in PBS for 5 minutes and dehydrated for 3 minutes each in 60%, 80% and 99,9% ethanol. The chromosome-specific repeat DXZ1 probe CEP X alpha satellite DNA labeled with spectrum green and DYZ1 probe CEP Y satellite III labeled with spectrum orange (Abbott Molecular) were used for this analysis. Hybridization mixtures containing both probes were prepared by mixing 1 part of the X-probe, 1 part of the Y-probe,

1 part of distilled water and 7 parts of hybridization buffer. Fifteen µl of hybridization mixture were added and covered by a 24 x 24 mm cover slip. The cover slips were sealed with rubber cement, and the DNAs denatured on a hot plate at 83,50C for 7 minutes and hybridized overnight in a humidified atmosphere at 420C. Hybridized slides were washed for 2 minutes at 730C in 0,4 x SSC with 0,3% Tween 20 and for 1 minute at room temperature in 2xSSC with 0,1% Tween 20. The slides were then mounted in Vectashield with DAPI.

Cells containing a red FISH signal located in a DAPI stained nucleus were identified by automatic scanning using two different types of scanners. The MDS (version 5.8.0) slide scanning system originally developed by Applied Imaging, and the MetaCyte scanning system developed by Metasystems. With the MDS scanning system, slides were scanned at 20X magnification using scan function 5. With MetaCyte, slides were scanned at 10x magnification using a classifier developed and optimized in-house for detection of true Spectrum Orange FISH signals. After scanning, cells identified by the scanner were inspected visually by automatic relocation. Cells that had one green X signal and one orange Y-signal significantly bigger than the X-signal were classified as male fetal cells.

Two times 98 CD105 positive male fetal cells identified by one XY FISH and two times 980 surrounding maternal cells are isolated by Laser Microdissection and Pressure Catapulting as described in example 1. The RNA of the two fetal samples and the two control samples is amplified by Miltenyi Biotec by their SuperAmp Service, and the amplified cDNAs is used for making two PIQOR™ Stem Cell Microarray (biological replicate), as described in example 1.

From the PIQOR microarray results 24 additional genes (table 2) are selected on the basis of 3 different criterions. First, the gene should show a high or relatively high fetal/maternal signal ratio in both PIQOR arrays made from CD105 positive fetal cells hybridized once (the biological replicate). In addition, the gene should show a high or relatively high fetal/maternal signal ratio in at least one of the two PIQOR arrays made from the 23 non-enriched fetal cells hybridized twice (the technical replicate). Finally, the gene should show very low or no expression in whole blood.

**Table 2**

| Gene Symbol | Accession number | Definition | Ratio: Fetal signal/Maternal signal (PIQOR arrays) |
|---|---|---|---|
| THBD (CD141) | NM_000361 | Homo sapiens thrombomodulin (THBD), mRNA. | 2.06 / 33 % |
| | | | 1.92 / 36 % |
| | | | 0.97 / 52 % |
| | | | 1.87 / 47 % |
| FLT3 (CD135) | NM_004119 | Homo sapiens fms-related tyrosine kinase 3 (FLT3), mRNA. | 2.32 / 30 % |
| | | | 1.83 / 11 % |
| | | | 1.92 / 58 % |
| | | | 2.00 / 21 % |
| F3 (CD141) | NM_001993 | Homo sapiens coagulation factor III (thromboplastin, tissue factor)(F3), mRNA. | 2.66 / 46 % |
| | | | 2.10 / 25 % |
| | | | 1.87 / 60 % |
| | | | 1.87 / 14 % |
| MCAM (CD146) | NM_006500 | Homo sapiens melanoma cell adhesion molecule (MCAM), mRNA. | 4.23 / 20 % |
| | | | 2.94 / 17 % |
| | | | 2.60 / 48 % |
| | | | 3.07 / 29 % |
| CD33 | NM_001772 | Homo sapiens CD33 molecule (CD33), transcript variant 1, mRNA. | 5.38 / 7 % |
| | | | 4.07 / 9 % |
| | | | 8.57 / 38 % |
| | | | 7.69 / 50 % |
| CD68 | NM_001040059 | Homo sapiens CD68 molecule (CD68), transcript variant 1 and transcript variant 2, mRNA. | 2.42 / 37 % |
| | NM_001251 | | 2.19 / 16 % |
| | | | 2.35 / 31 % |
| | | | 2.00 / 34 % |
| CDH5 (CD144) | NM_001795 | Homo sapiens cadherin 5, type 2 (vascular endothelium) (CDH5), mRNA. | 0.69 / - % |
| | | | 0.71 / - % |
| | | | 1.84 / 36 % |
| | | | 2.00 / 104 % |
| SELE (CD62E) | NM_000450 | Homo sapiens selectin E (SELE), mRNA. | 1.43 / 16 % |
| | | | 2.11 / 24 % |
| | | | 3.51 / 53 % |
| | | | 2.98 / 27 % |
| GJA5 (GAP) | NM_181703 | Homo sapiens gap junction protein, alpha 5, 40kDa (GJA5), transcript variant B, mRNA. | 1.95 / 45 % |
| | | | 1.82 / 21 % |
| | | | 0.97 / 49 % |
| | | | 1.24 / 39 % |
| ITGA5 | NM_002205 | Homo sapiens integrin, alpha 5 (fibronectin receptor, alpha polypeptide) (ITGA5), mRNA. | 3.25 / 33 % |
| | | | 2.51 / 17 % |
| | | | 1.88 / 53 % |
| | | | 2.25 / 56 % |
| KCNK3 (TASK-1) | NM_002246 | Homo sapiens potassium channel, subfamily K, member 3 (KCNK3), mRNA. | |
| | | | 2.04 / 37 % |
| | | | 1.90 / 28 % |
| | | | 1.73 / 40 % |
| CNTFR | NM_001842 | Homo sapiens ciliary neurotrophic factor receptor (CNTFR),transcript variant 1 and transcript variant 2, mRNA. | |
| | NM_147164 | | 3.31 / 28 % |
| | | | 2.62 / - % |
| | | | 3.49 / 53 % |
| F11R (JAM-1) | NM_016946 | Homo sapiens F11 receptor (F11R), mRNA. | 4.43 / 30 % |
| | | | 3.56 / 22 % |
| | | | 2.52 / 3 % |
| | | | 3.32 / 55 % |
| SDC4 (Syndecan 4) | NM_002999 | Homo sapiens syndecan 4 (SDC4), mRNA. | |
| | | | 2.01 / 11 % |
| | | | 1.73 / 40 % |
| | | | 2.19 / 19 % |
| ACVR2B | NM_001106 | Homo sapiens activin A receptor, type IIB (ACVR2B), mRNA. | 1.90 / 0 % |
| | | | 1.48 / 9 % |
| | | | 1.39 / 41 % |
| | | | 1.74 / 27 % |
| ACVR2A | NM_001616 | Homo sapiens activin A receptor, type IIA (ACVR2A), mRNA. | 1.30 / 17 % |
| | | | 2.08 / 23 % |
| | | | 2.60 / 44 % |
| | | | 1.76 / 30 % |
| CD44 (CD44v6+ CD44v4/5) | NM_000610 | Homo sapiens CD44 molecule (Indian blood group) (CD44), transcript variant 1 and transcript variant 2, mRNA. | 1.30 / 24 % |
| | NM_001001389 | | 3.23 / 77 % |
| | | | 7.09 / 75 % |
| | | | 1.96 / 48 % |
| STX1A | NM_004603 | Homo sapiens syntaxin 1A (brain) (STX1A), mRNA. | |
| | | | 5.09 / 26 % |
| | | | 4.23 / 62% |
| | | | 4.80 / 55 % |
| TEK (Tie2) | NM_000459 | Homo sapiens TEK tyrosine kinase, endothelial (TEK), mRNA. | |
| | | | 2.13 / 31 % |
| | | | 2.23 / 55 % |
| | | | 2.37 / 65 % |
| L1CAM (CD171) | NM_000425 | Homo sapiens L1 cell adhesion molecule (L1 CAM), transcript variant 1 and transcript variant 2, mRNA. | 1.98 / 35 % |
| | NM_024003 | | 2.37 / 22 % |
| | | | 2.37 / 61 % |
| | | | 2.18 / 28 % |
| NCAM1 (CD56) | NM_000615 | Homo sapiens neural cell adhesion molecule 1 (NCAM1), transcript variant 1, transcript variant 2 and transcript variant 3, mRNA. | 0.90 / - % |
| | NM_001076682 | | 1.60 / 17 % |
| | NM_181351 | | 3.83 / 54 % |
| | | | 2.10 / 27 % |
| ALPP | NM_001632 | Homo sapiens alkaline phosphatase, placental (Regan isozyme) (ALPP), mRNA | 1.63 / - % |
| | | | 1.83 / 13 % |
| | | | 3.10 / 52 % |
| | | | 1.70 / 93 % |
| ALPPL2 | NM_031313 | Homo sapiens alkaline phosphatase, placental-like 2 (ALPPL2), mRNA. | 1.16 / 82 % |
| | | | 5.46 / 44 % |
| | | | 7.91 / 22 % |
| | | | 2.26 / 61 % |
| MMP23B (and MMP23A) | NM_006983 | Homo sapiens matrix metallopeptidase 23B (MMP23B), mRNA. Homo sapiens matrix metallopeptidase 23A (pseudogene) (MMP23A),non-coding RNA. | 0.86 / 52 % |
| | NR_002946 | | 1.11 / 56 % |
| | | | 2.80 / 67 % |
| | | | 2.05 / 54 % |

### Items

1. A method comprising the steps of
   a. Providing a maternal blood sample or a fraction thereof
   c. Contacting the sample with
      i. a hybridization probe directed to a nucleic acid comprising at least 10 bp of a gene selected from the group consisting of TMEFF2, ABHD2, ACVR2B, ADAM11, AHNAK, AK000420, ALS2CL, BC089454, BC111482,CB123670, CCNA2, CD7, CPS1, CRYL1, D4ST1, DKFZp434F142, EDN1, EEF1A1, ENST00000356196, FLJ35740, FYN, GCC2, GSK3A, HIST1H2AJ, HLA-C,HMGA1, HMGN2, ITGA7, KCNK4, KMO, KY, LOC389286, MAD2L1, MAPK3, MYL6, NBR2, NTRK1, PF4, PGK1, PPIA, QPRT, RGPD2, RP11-78J21.1, RPL23, RPL23A, RPL26, RPL39, RPS27, SLAMF1, SYT9, T (BRACHYURY), THC2265980, THC2274391, TP73, TPM3, UBL4A, WRN, ZFYVE9, ZNF283, ZNF539, ZNF614, CD141, CD135, CD142/F3, CD146, CD33, CD68, CD144, CD62E, GAP/GJA5, ITGA5, KCNK3/TASK-1, CNTFR, JAM-1, SDC4/Syndecan 4, ACVR2B, ACVR2A, CD44v6+CD44v4/5, STX1A, TEK/Tie2, CD171, NCAM/CD56, ALPP, ALPPL2 and MMP23A/MMP23B or
      ii. a ligand directed to an antigen encoded by a gene selected from the group consisting of TMEFF2, ABHD2, ACVR2B, ADAM11, AHNAK, AK000420, ALS2CL, BC089454, BC111482,CB123670, CCNA2, CD7, CPS1, CRYL1, D4ST1, DKFZp434F142, EDN1, EEF1A1, ENST00000356196, FLJ35740, FYN, GCC2, GSK3A, HIST1H2AJ, HLA-C,HMGA1, HMGN2, ITGA7, KCNK4, KMO, KY, LOC389286, MAD2L1, MAPK3, MYL6, NBR2, NTRK1, PF4, PGK1, PPIA, QPRT, RGPD2, RP11-78J21.1, RPL23, RPL23A, RPL26, RPL39, RPS27, SLAMF1, SYT9, T (BRACHYURY), THC2265980, THC2274391, TP73, TPM3, UBL4A, WRN, ZFYVE9, ZNF283, ZNF539, ZNF614, CD141, CD135, CD142/F3, CD146, CD33, CD68, CD144, CD62E, GAP/GJA5, ITGA5, KCNK3/TASK-1, CNTFR, JAM-1, SDC4/Syndecan 4, ACVR2B, ACVR2A, CD44v6+CD44v4/5, STX1A, TEK/Tie2, CD171, NCAM/CD56, ALPP, ALPPL2 and MMP23A/MMP23B
2. The method of item 1, further comprising a step of identifying fetal cells of the sample or a step of enriching fetal cells of the sample.
3. The method of any of items 1 and 2, wherein identification comprises detecting the presence of the ligand or the hybridization probe on or in the fetal cells.
4. The method of any of items 1 and 2, wherein enrichment comprises contacting the maternal blood sample with a ligand directed to a cell surface exposed antigen encoded by a gene selected from the group consisting of tomoregulin, EDN1, AHNAK, , CD 105, SYT 9, KMO, HMGA1, Mouse T, ACVR2B, CD141, CD135, CD142/F3, CD146, CD33, CD68, CD144, CD62E, GAP/GJA5, ITGA5, KCNK3/TASK-1, CNTFR, JAM-1, SDC4/Syndecan 4, ACVR2B, ACVR2A, CD44v6+CD44v4/5, STX1A, TEK/Tie2, CD171, NCAM/CD56, ALPP, ALPPL2 and MMP23A/MMP23B.

## Claims

1. A method of detecting a fetal cell comprising the steps of
a. Providing a maternal blood sample or a fraction thereof
b. Fixating the cells
c. Contacting the sample with
i. a hybridization probe directed to a nucleic acid comprising at least 10 bp of a gene differentially expressed in fetal cells of a maternal blood sample selected from the group consisting of tomoregulin, EDN1, AHNAK, CD 105, SYT 9, KMO, HMGA1, Mouse T, ACVR2B, CD141, CD135, CD142/F3, CD146, CD33, CD68, CD144, CD62E, GAP/GJA5, ITGA5, KCNK3/TASK-1, CNTFR, JAM-1, SDC4/Syndecan 4, ACVR2B, ACVR2A, CD44v6+CD44v4/5, STX1A, TEK/Tie2, CD171, NCAM/CD56, ALPP, ALPPL2 and MMP23A/MMP23B or
ii. a ligand directed to an antigen encoded by a gene differentially expressed in fetal cells of a maternal blood sample selected from the group consisting of tomoregulin, EDN1, AHNAK, CD 105, SYT 9, KMO, HMGA1, Mouse T, ACVR2B, CD141, CD135, CD142/F3, CD146, CD33, CD68, CD144, CD62E, GAP/GJA5, ITGA5, KCNK3/TASK-1, CNTFR, JAM-1, SDC4/Syndecan 4, ACVR2B, ACVR2A, CD44v6+CD44v4/5, STX1A, TEK/Tie2, CD171, NCAM/CD56, ALPP, ALPPL2 and MMP23A/MMP23B

2. The method of claim 1, further comprising a step of identifying fetal cells of the sample or a step of enriching fetal cells of the sample.

3. The method of any of claims 1 and 2, wherein identification comprises detecting the presence of the ligand or the hybridization probe on or in the fetal cells.
